# EUROPEAN PATENT APPLICATION

(11) **EP 1 938 792 A1**
(43) Date of publication of application: **02.07.2008**
(21) Application number: 06425868.4
(22) Date of filing: 29.12.2006
(51) Int. Cl.: A61K 8/44, A61K 8/55, A61K 8/92, A61Q 19/00, A61Q 19/06

(54) **Cosmetic complex compound**

(71) Applicant: Vanity Line S.p.A., 15076 Ovada, AL (IT)
(72) Inventor: Saverio, Antonaccio, 15076 Ovada (AL) (IT)
(74) Representative: Garavelli, Paolo

(57) **Abstract**

A cosmetic complex compound is disclosed, comprising phosphatidylcoline, carnitine of a lipo-soluble form, ozonised oil and sunflower oil.

## Description

The present invention refers to a cosmetic complex compound, used in particular for the topic application or the formulation of topically-active cosmetic preparations active in the lipo-reduction and against the formation of lipodystrophic symptomatologies (cellulitis).

Several studies have demonstrated the capability of phosphatidylcholine (a derivative of soya lecithin) to reduce the systemic levels of cholesterol and triglycerides, in addition to increase the sensitivity of membrane receptors to insulin, increasing the lipolysis. Phosphatidylcholine in fact is a natural emulsifying agent that solubilises fats by penetrating inside the adipocyte itself and "squeezing" it. Moreover, and above all, it operates as an enzymatic activator for lipolytic enzimes that generates the probably definitive reduction of adipocytes.

Phosphatidylcoline also resulted in a particularly interesting mixture to activate the transport of active principles and drugs and to promote their cutaneous penetration, such behaviour being validated by numerous studies (among the others: Korting H. C. et al: "Anti-inflammatory activity of hamamelis distillate applied topically to the skin; influence of vehicle and dose" - Eur J Clin Pharmacol, 44, 315-318, 2000; Chinhan Kim et al: "The skin permeation enhancing effect of phosphatidylcoline: caffeine as a model of active" - J of Cosmet Sci, 53, 363-374, 2002): Phosphatidylcoline is thereby to be preferred, in certain cosmetic and pharmaceutical preparations, to other emulsifying agents that could damage the lipidic barrier, forming mycelia that are inserted in its continuos structure, opening it and making it easier for foreign substances to penentrate inside the skin and the skin water to go out therefrom. It is therefore known that cosmetic preparations containing phosphatidylcoline improve the skin barrier function and reduce water dispersion transcutaneously.

Carnitine instead is a compound that derives from animal food, such as meat and fish, or from cheese products; it can also be synthetised by the human organism in liver and kidneys starting from glutammate and two essential aminoacids such as methionine and lysine in the presence of vitamins B₆, C and iron. Its role in the organism is binding and allowing the passage of fat acids (as acetylCoA-carnitine) through mitochondria membranes, thereby making them available for β-oxidation. Since carnitine facilitates the passage of long-chain lipids inside mitochondria, and since lipids oxidation produces energy, it is generally used for increasing cardiovascular efficiency and muscular strength, to delay the fatigue sensation and reduce muscular pain.

The topic application of ozonised oil instead, generally obtained by gurgling a mixture of ozone and oxygen in olive or sunflower oil, determines an increase of lipids metabolism, intervening on fats demolition and a direct lipolitic effect, with an increase of energy production. In fact it directly reacts with unsaturated fat acids determining, in an acquous environment, the formation of peroxides, by fragmenting lipidic chains that assume an hydrophilic character. In this way, their disposal is enabled through lymphatic discharge routes.

Though the validity of the present invention is anyway not linked to the validity of the above described action mechanisms, the Applicant has surprisingly discovered that the synergy of components with a strong lipolitic function, such as phosphatidylcoline and ozonised oil, allows obtaining very good results both in reducing adiposity (due to the action of the phosphatidylcoline) and in toning-up the tissues (due to the action of the ozonised oil); the lipolitic action is further increased by the simultaneous presence of phosphatidylcoline, ozonised oil and carnitine, since their effects, as it has further been discovered, are mutually reinforcing.

The known art however does not propose complexes or compounds for pharmaceutical or cosmetic use that provide for the use, in mutual composition, of phosphatidylcoline, carnitine and ozonised oil for preparing cosmetic formulations operating as liporeducers and against the formation of cellulitis, or for their topic cutaneous application.

Therefore, object of the present invention is solving the above prior art problems by providing a cosmetic complex compound characterised by the composition and the synergic topic action of phosphatidylcoline, ozonising oil and carnitine.

The above and other objects and advantages of the invention, as will appear from the following description, are obtained with a cosmetic complex compound as claimed in claim 1. Preferred embodiments and non-trivial variations of the present invention are the subject matter of the dependent claims.

The present invention will be better described by some preferred embodiments thereof, provided as a non-limiting example.

The cosmetic complex compound according to the present invention is a composition comprising phosphatidylcoline, carnitine in a lipo-soluble form, ozonised oil and sunflower oil. In particular, the cosmetic complex compound according to the present invention comprises:
- a weight concentration preferably included between 10% and 15%, based on the total weight of the complex compound, of phosphatidylcoline (INCI denomination): in particular, the weight concentration of phosphatidylcoline is still more preferably equal to 12% based on the total weight of the complex compound;
- a weight concentration preferably included between 50% and 70%, based on the total weight of the complex compound, of carnitine in a lipo-soluble form (INCI denomination: glycerin, carnitine linoleate, aqua): in particular, the weight concentration of carnitine in a lipo-soluble form is still more preferably equal to 64% based on the total weight of the complex compound;
- a weight concentration preferably included between 0.5% and 1,0%, based on the total weight of the complex compound, of ozonised oil (INCI denomination: ozonized sunflower seed oil): in particular, the weight concentration of ozonised oil is still more preferably equal to 0,8% based on the total weight of the complex compound; and
- the remaining balance to 100%, based on the total weight of the complex compound, of sunflower oil (INCI denomination: helianthus annuus); in particular, the weight concentration of ozonised oil is preferably included between 14% and 39.5% based on the total weight of the complex compound.

The following non-limiting example of preparation will further describe the invention.

### Preparation Example 1

To 12 g of phosphatidylcoline, 64 g of carnitine in a soluble form and 0.8 g of ozonised oil are added; afyer homogenisation of the compound through mixing, 23.2 g of sunflower oil are added, keeping on mixing. 100 g of cosmetic complex compound according to the present invention are obtained.

Obviously, the cosmetic complex compound according to the present invention can be used both for its direct topic application and in the formulation of cosmetic preparations with active topic action in lipo-reduction and against the formation of lipodystrophic symptomatologies (cellulitis).

Example of suitable topic application embodiments of the complex compound according to the present invention comprise creams, gels, ointments, lotions and other commonly used forms: in fact, plasters, gauzes, pads or pieces of clothing can also be provided, drenched with the complex compound according to the present invention. The modes for administering the complex compound according to the present invention, obviously in case of its direct topic use, will depend on the particular form chosen and the type of application.

The complex compound according to the present invention could obviously further comprise other known active principles, with complementary or anyway useful action for the provided cosmetic uses, without thereby departing from the scope of the present invention. For example, the complex compound of the invention could include vitamins, aminoacids, vegetable extracts, emollients, anti-bacterial agents, topic anfi-flammatory agents, etc.

In order to verify the actual efficiency of the cosmetic complex compound according to the present invention and experiment its tolerability, the Applicant has performed a series of tests on voluntary human samples according to the following modes. In the experimentation, ten voluntary women have been selected, whose age was over 18 years (in particular with age included between 27 and 45 years) affected by localised adiposity and edematic fibro-sclerotic panniculopathy of lower limbs. After a systematic application for fifty-six days of a reducing emulsion containing the complex compound according to the present invention on each one of the women, the results obtained from the experiments were as follows:
- no one of the women showed intolerance phenomena when using the preparation;
- the judgment given by the women about the stretching of the cosmetic complex compound according to the present invention has been optimum in three cases, good in six cases, discrete in one case;
- the judgment given by the women on the absorption of the cosmetic complex compound according to the present invention has been optimum in one case, good in six cases, discrete in three cases;
- the global efficiency judgment has been favourable in eight cases, scarce in two cases.

The following Table 1 schematically shows the results of measures performed by the Applicant on the circumference of the thighs of the ten women at different times (immediately before starting the experimentation, after 28 days and after 56 days from the beginning of the experimentation) of the experimentation and the treatment through the cosmetic complex compound according to the present invention, from which a real efficiency is detected in lipo-reduction and against the formation of lipodystrophic situations:

**Table 1 : circumferences of volunteers' tighs during the experimentation**

| Volunteer | Age | Right thigh (cm) | Left thigh (cm) | Average value (cm) | Right thigh (cm) | Left thigh (cm) | Average value (cm) | Right thigh (cm) | left thigh (cm) | Average value (cm) |
|---|---|---|---|---|---|---|---|---|---|---|
| **Nr.** | **Years** | **Beginning** | **Beginning** | **right and left** | **after 28 days** | **after 28 days** | **right and left** | **after 56 days** | **after 56 days** | **right and left** |
| 1 | 37 | 57,2 | 56 | 56,6 | 57,2 | 56 | 56,6 | 56,2 | 55 | 55,6 |
| 2 | 27 | 53,5 | 52,6 | 53,05 | 53,4 | 52,6 | 53 | 53,4 | 52,5 | 52,95 |
| 3 | 33 | 57 | 57,2 | 57,1 | 56 | 56,1 | 56,05 | 55,7 | 56 | 55,85 |
| 4 | 45 | 61,3 | 61,8 | 61,55 | 60,5 | 61,4 | 60,95 | 60,1 | 60,8 | 60,45 |
| 5 | 30 | 53,6 | 53,2 | 53,4 | 53 | 52,4 | 52,7 | 52,7 | 52,4 | 52,55 |
| 6 | 42 | 52,4 | 52,5 | 52,45 | 52,2 | 52,4 | 52,3 | 51,5 | 51,8 | 51,65 |
| 7 | 41 | 54,8 | 53,3 | 54,05 | 54,9 | 53,5 | 54,2 | 54,9 | 53,6 | 54,25 |
| 8 | 42 | 50,9 | 51,2 | 51,05 | 50,3 | 50,5 | 50,4 | 50,1 | 49,8 | 49,95 |
| 9 | 34 | 53,2 | 53,4 | 53,3 | 53,1 | 53,2 | 53,15 | 52,9 | 52,9 | 52,9 |
| 10 | 28 | 53,3 | 53,2 | 53,25 | 52,2 | 52,1 | 52,15 | 52 | 51,9 | 51,95 |

## Claims

1. Cosmetic complex compound, **characterised in that** it comprises phosphatidylcoline, carnitine in a lipo-soluble form, ozonised oil and sunflower oil.

2. Cosmetic complex compound according to claim 1, **characterised in that** it comprises a weight concentration of said phosphatidylcoline included between 10% and 15% based on the total weight of the complex compound.

3. Cosmetic complex compound according to claim 1 or 2, **characterised in that** it comprises a weight concentration of said phosphatidylcoline equal to 12% based on the total weight of the complex compound.

4. Cosmetic complex compound according to claim 1, **characterised in that** it comprises a weight concentration of said carnitine in a lipo-soluble form included between 50% and 70% based on the total weight of the complex compound.

5. Cosmetic complex compound according to claim 1 or 4, **characterised in that** it comprises a weight concentration of said carnitine in a lipo-soluble form equal to 64% based on the total weight of the complex compound.

6. Cosmetic complex compound according to claim 1, **characterised in that** it comprises a weight concentration di said ozonised oil included between 0.5% and 1.0% based on the total weight of the complex compound.

7. Cosmetic complex compound according to claim 1 or 6, **characterised in that** it comprises a weight concentration of said ozonised oil equal to 0.8% based on the total weight of the complex compound.

8. Cosmetic complex compound according to any one of the previous claims, **characterised in that** it comprises a weight concentration of said sunflower oil included between 14% and 35,5% based on the total weight of the complex compound.

9. Use of the cosmetic complex compound according to the previous claims for a topic application.

10. Use of the cosmetic complex compound according to any one of claims 1 to 8 for formulating cosmetic preparations.
